# EUROPÄISCHE PATENTSCHRIFT

(11) **EP 0 945 451 B1**
(45) Veröffentlichungstag und Bekanntmachung des Hinweises auf die Patenterteilung: **18.12.2002**
(21) Anmeldenummer: 99106019.5
(22) Anmeldetag: 25.03.1999
(51) Int. Cl.: C07D 311/92, C07D 407/04, G02B 5/23

(54) **Photochrome Diaryl-Naphthopyrane mit zumindest einem aminosubstituierten Arylrest**
Photochrome diaryl-naphtopyran with at least one aminosubstituted aryl rest
Diarylnaphtopyranne photochrome avec au moins un reste aryle amino substitué

(30) Priorität: 25.03.1998 DE 19820781
(43) Veröffentlichungstag der Anmeldung: 29.09.1999
(73) Patentinhaber: Optische Werke G. Rodenstock, 80469 München (DE)
(72) Erfinder: Mann, Claudia Dr., 80634 München (DE); Melzig, Manfred Dr., 82234 Wessling (DE); Weigand, Udo Dr., 80638 München (DE)
(74) Vertreter: Münich, Wilhelm, Dr.

(56) Entgegenhaltungen:
- EP-A- 0 250 193
- WO-A-98/04973
- WO-A-98/45281
- US-A- 5 238 981
- US-A- 5 658 500
- CHEMICAL ABSTRACTS, vol. 127, no. 3, 1997 Columbus, Ohio, US; abstract no. 42375x, Seite 1327; XP002070792 & JP 09 124645 A (TOKUYAMA)

## Beschreibung

Die Erfindung betrifft photochrome Diaryl-Naphthopyrane mit zumindest einem aminosubstituerten Arylrest. Es sind photochrome Verbindungen, die in o-Stellung zum Sauerstoffatom des Pyransystems einen entsprechend mit einer Aminogruppe substituierten Phenyl- oder Naphthylrest besitzen.

### Stand der Technik

Pyrane, speziell Naphthopyrane und von diesen abgeleitete größere Ringsysteme, sind die derzeit am meisten bearbeitete Klasse photochromer Verbindungen. Obwohl bereits im Jahr 1966 erstmals zum Patent angemeldet (US 3.567.605), konnten erst in den 90er Jahren Verbindungen entwickelt werden, die für den Einsatz in Brillengläsern geeignet erschienen. Eine von den Pyranen geeignete Verbindungsklasse sind zum Beispiel die 3,3-Diaryl-3H-naphtho[2,1-b]pyrane, die fast durchweg in angeregter Form eine gelbe, orange oder rotorange Farbe zeigen.

Es sind zahlreiche Veröffentlichungen bekannt, in denen versucht wurde, die Eigenschaften der 3,3-Diaryl-3H-naphtho[2,1-b]pyrane zu modifizieren:

Beispielsweise sind in der US 5.066.818 Verbindungen beschrieben, bei denen in die Arylreste in o-Stellung zur Verknüpfung mit dem Pyran Substituenten eingeführt wurden. Aminogruppen sind in dieser Druckschrift nicht aufgeführt.

In der US 5.238.981 soll durch zusätzliche Substitution in 8-Stellung des Naphthopyransystems eine bathochrome Verschiebung und intensivere Eindunkelung erreicht werden. Hierbei können die Arylreste auch mit Di-(C₁-C₅)-Alkylaminogruppen substituiert sein. Die 4-Dimethylamino-Verbindung ergibt eine rote Färbung, wobei aber im Polymer nur die geöffnete Form des Moleküls vorliegt, die nicht photochrom reagiert.

Ferner werden in der US 5.244.602 zum Beispiel Substituenten in 5-Stellung des Naphthopyransystems und in der US 5.274.132 vinyloge Arylreste beschrieben; allerdings sind Aminogruppen als Substituenten an den Arylresten nicht aufgeführt.

Die US 5.369.158 ist als Teilanmeldung aus der US 5.238.981 hervorgegangen und entspricht deren Offenbarung nahezu vollständig, wobei zusätzlich auch heteroaromatische Ringe in 3,3-Stellung beschrieben sind, wie beispielsweise der Pyridinring.

Aus der US 5.384.077 sind Verbindungen bekannt, in denen einer der beiden Arylreste in 3-Stellung ein sauerstoffhaltiger Heterocyclus sein kann. Der carbocyclische Arylrest kannzudem mit -NH₂, Mono- oder Di-(C₅-C₇)-Alkylaminogruppen substituiert sein. Nach den angebenen λₘₐₓ-Werten handelt es sich um gelborange Verbindungen. Die erfindungsgemäßen Aminosubstituenten werden nicht erwähnt.

In einer weiteren Druckschrift, der US 5.637.262, ist in 3-Stellung als Heteroaromat auch Pyridyl beschrieben. Als mögliche Substituenten der Aryl- bzw. Heteroarylgruppen werden auch (C₁-C₅)-Dialkylaminogruppen genannt. Weitere Hinweise zu diesen Gruppierungen sind der Druckschrift nicht zu entnehmen.

In der US 5.578.252 werden 3H-Naphtho[2,1-b]pyrane offenbart, die in 8-Position einen sauerstoffhaltigen Substituent (z.B. Methoxy) und entweder eine Alkylgruppe in der 7-Position oder eine Carbonylgruppe in der 9-Position besitzen (a.a.O., Sp. 2, Z. 19-25). Diese Verbindungen sollen über gelb nach orange und rot reichende Farben zeigen (a.a.O., Sp. 9, Z. 24-27). Die dort angegebene sehr umfangreiche Substituentenliste an den Aryl- oder Heteroarylgruppen führt neben Amino, Mono- und Di-(C₁-C₆)-Alkylaminogruppen auch andere Amine wie Morpholin, Piperidin, 1-Indolinyl etc. auf. In der Beschreibung finden sich keinerlei Angaben über diese Verbindungen bzw. ihre Verwendbarkeit.

In den genannten Druckschriften werden die erfindungsgemäßen Aminosubstituenten an den Aryl- oder Heteroarylgruppen in 3-Stellung der 3,3-Diaryl-3H-naphtho[2,1-b]pyrane nur in der US 5.578.252 unter einer großen Reihe zahlreicher anderer Substituentengruppen am Rande erwähnt, wobei jegliche näheren Angaben über Eigenschaften und Verwendbarkeit fehlen.

Bei den aufgeführten Verbindungsklassen sind insbesondere auch die 2,2-Diaryl-2H-naphtho[1,2-b]pyrane von Interesse, da diese Verbindungen bekanntermaßen gegenüber den analogen 3,3-Derivaten im geöffneten Zustand eine deutliche bathochrome Verschiebung aufweisen. Nachteilig ist aber, daß diese bekannten Verbindungen in der Regel unakzeptabel langsam in ihrer Aufhellung sind, sofern sie nicht in 5-Stellung des Naphthopyransystems entsprechend substituiert werden.

Von den 2,2-Diaryl-2H-naphtho[1,2-b]pyranen ist folgender relevanter Stand der Technik bereits bekannt:

In der US 5.573.712 sind derartige Verbindungen beschrieben, wobei als Substituenten in 2-Stellung heteroaromatische Gruppen, wie Pyridin, und substituierte und unsubstituierte Arylgruppen aufgeführt sind; es werden aber keine Aminosubstituenten erwähnt. Als weitere sich direkt am Naphthopyransystem befindende Substituenten sind Indolin-5-yl und 1,2,3,4-Tetrahydronaphth-6-yl (THN) genannt, die aber nicht explizit beschrieben sind.

Die US 5.651.923 beschreibt ebenfalls längerwellig absorbierende Naphthopyransysteme, die auch durch an die Naphthalinringe ankondensierte heterocyclische Ringsysteme gebildet werden. Die in 2-Stellung möglichen Aryl- und Heteroarylgruppen können Amino-, Mono- und Di-(C₁-C₆)-Alkylaminogruppen, Morpholin, Piperidin, 1-Indolinyl, Pyrrolidyl, 1-Imidazolidyl, 2-lmidazolin-1-yl, 2-Pyrazolidyl, Pyrazolinyl und 1-Piperazinyl darstellen, ferner sind auch die direkt am Naphthopyransystem gebundenen vorstehend genannten bicyclischen N-haltigen Systeme 5-lndolinyl und 6-THN als Substituenten beschrieben. Diese Amine werden in der Beschreibung nicht näher erläutert.

In den US 5.650.098, US 5.656.206 und US 5.658.501 werden die gleichen Substituenten wie zuvor offenbart, wobei nicht weiter auf die genannten Verbindungen eingegangen wird. Es handelt sich hierbei um in 5-Stellung verschieden substituierte Naphthopyrane. Allein in der US 5.658.500 finden sich Einzelheiten und experimentelle Daten zu drei Morpholinverbindungen (vgl. a.a.O., Bespiele 1, 2 und 3).

### Hintergrund der Erfindung

Die vorliegende Erfindung hat daher die Aufgabe neue photochrome Verbindungen bereitzustellen, die verbesserte Eigenschaften gegenüber den im Stand der Technik beschriebenen Strukturen besitzen. Darüber hinaus sollten die Verbindungen keine ausgeprägte basische Funktion besitzen, da diese erfahrungsgemäß sowohl bei der Herstellung von photochromen Kunststoffgläsern im Massefärbungsverfahren leicht Protonen addieren und Farbabweichungen ergeben als auch im Dauergebrauch unter Lichteinwirkung zu Nebenreaktionen neigen, die zu einem Verlust der photochromen Reaktion führen.

Ferner soll ein Verfahren zur Herstellung der neuen Verbindungen zur Verfügung gestellt werden, wodurch die Verbindungen bei verhältnismäßig einfacher Verfahrensführung in guten Ausbeuten erhalten werden.

Die oben genannte Aufgabe wurde gelöst durch Bereitstellung von photochromen Diaryl-Naphthopyranen mit den nachfolgenden allgemeinen chemischen Strukturformeln (I) oder (II): 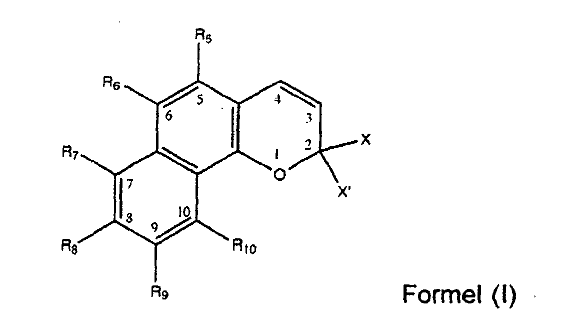 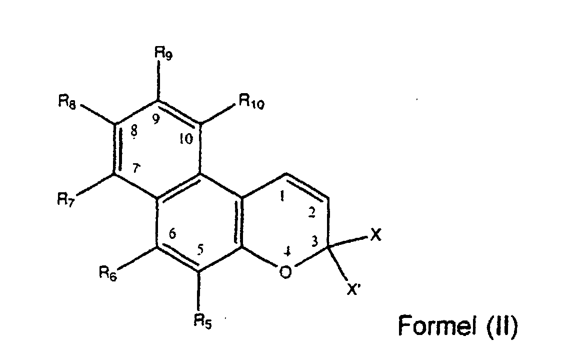 wobei R₅ bis R₁₀ unabhängig voneinander gleich oder verschieden sind und aus der Gruppe Z, bestehend aus H, OH, C₁-C₁₀-Alkyl, C₁-C₆-Alkoxy, C₅-C₇-Cycloalkyl, Phenyl, Benzyl, Allyl, C₁-C₆-Alkoxy-( C₁-C₄)-Alkyl, Mono- oder Di-(C₁-C₆)-Alkyl substituiertem Phenyl, Furyl, Thienyl, Mono- oder Di-(C₁-C₆)-Alkoxy substituiertem Phenyl, C₁-C₆-Haloalkyl und Halogen ausgewählt sind, wobei das Halogen F, Cl oder Br und R₅ kein Wasserstoff ist;
X und X' unabhängig voneinander gleich oder verschieden sind und ausgewählt sind aus einer der folgenden Gruppen:
- Gruppe (1):: unsubstituierten, mono-, di- oder trisubstituierten Arylgruppen, Phenyl oder Naphthyl, wobei die Arylsubstituenten ausgewählt sind aus der Gruppe Z;
- Gruppe (2):: mono-, di- oder trisubstituierten Arylgruppen Phenyl oder Naphthyl, wobei zumindest ein Substituent ausgewählt ist aus der Gruppe A, bestehend aus cyclischen Aminosubstituenten, wie Morpholin, Piperidin, Pyridin, Pyrrol, Pyrazol, Pyrazolidin, Piperazol, Indolin, Imidazol, Phenothiazin, Phenoxazin, Phenazin, Acridin, Carbazol, die unsubstituiert, mono-, di- oder trisubstituiert sind und der oder die Substituenten ausgewählt sind aus der Gruppe Z, und jeder weitere Substituent ausgewählt ist aus der Gruppe Z; oder
- Gruppe (3):: mono-, di- oder trisubstituierten Arylgruppen, Phenyl oder Naphthyl, wobei zumindest ein Substituent ausgewählt ist aus der Gruppe A', bestehend aus Aminodiphenyl, wobei ein oder zwei Phenylringe unabhängig voneinander unsubstituiert, mono-, di- oder trisubstituiert sind, und die Substituenten ausgewählt sind aus der Gruppe Z,
und entweder X oder X' aus einer der oben definierten Gruppen (2) oder (3) ausgewählt ist, mit der Maßgabe, daß bei Verbindungen mit der Strukturformel (I) die cyclischen Aminosubstituenten Pyridin, Morpholin, Piperidin, 1-Indolinyl, Pyrrolidyl, 1-Imidazolidyl, 2-Imidazolin-1-yl, 2-Pyrazolidyl, Pyrazolinyl und 1-Piperazinyl aus der Gruppe (2) ausgeschlossen sind.

Gemäß einer vorteilhaften Ausführungsform der vorliegenden Erfindung sind in den photochromen Diaryl-Naphthopyranen der oben dargestellten Formeln (I) und (II) R₅ bis R₁₀ unabhängig voneinander gleich oder verschieden und aus der oben definierten Gruppe Z ausgewählt; X oder X' sind ausgewählt aus der Gruppe, bestehend aus unsubstituierten, mono-, di- oder trisubstituierten Arylgruppen, Phenyl oder Naphthyl, wobei die Arylsubstituenten aus der Gruppe, bestehend aus OH,C₁-C₁₀-Alkyl, C₁-C₆-Alkoxy und Halogen und das verbliebene X oder X' aus einer der Gruppen (2) oder (3) mit der oben genannten Einschränkung ausgewählt ist.

Gemäß einer weiteren erfindungsgemäßen Ausführungsform sind R₅ bis R₁₀ unabhängig voneinander gleich oder verschieden und aus der oben definierten Gruppe Z ausgewählt, und X und X' sind beide aus einer der Gruppen (2) oder (3) mit der oben genannten Einschränkung ausgewählt.

Nach einer weiteren bevorzugten Ausführungsform der Erfindung sind X und X' aus der gleichen Gruppe ausgewählt, insbesondere sind X und X' gleich.

Selbstverständlich umfassen die im Rahmen der Erfindung beschriebenen Substituenten, die in die erfindungsgemäßen Verbindungen eingeführt werden, auch die dem Fachmann auf diesem Gebiet bekannten bathochrom wirkenden Gruppierungen. Derartige Substitutionen, wie beispielsweise die Einführung einer Methoxygruppe in 8-Stellung des Naphthopyransystems am Naphthalingerüst wirken additiv.

Gegenstand der vorliegenden Erfindung sind auch die photochromen 2,2-Diphenyl-2H-naphtho[1,2-b]pyrane mit der nachfolgenden chemischen Strukturformel (III): 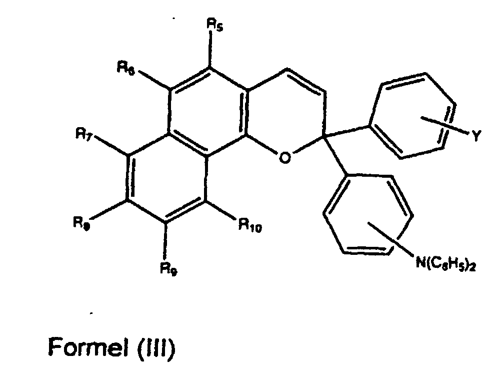 sowie die photochromen 3,3-Diphenyl-3H-naphtho[2,1-b]pyrane mit den nachfolgenden chemischen Strukturformeln (IV) oder (V): 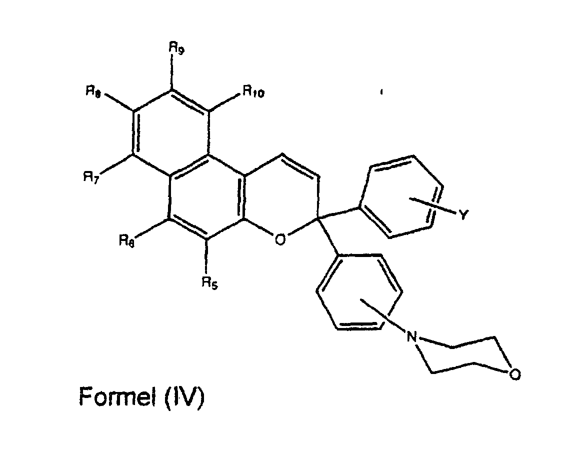 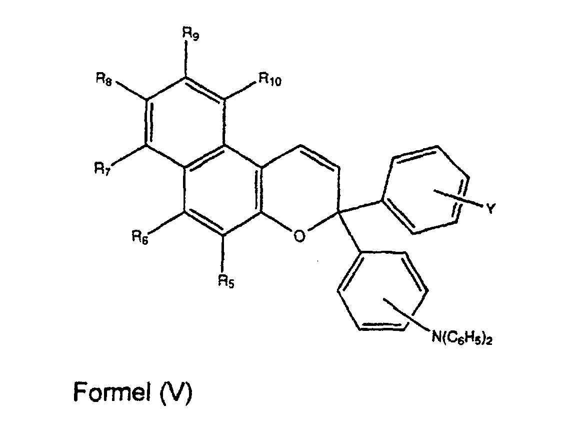 wobei
R₅ bis R₁₀ ausgewählt sind aus der oben definierten Gruppe Z und Y ausgewählt ist aus der Gruppe der Halogene F, Cl und Br.

In einer weiteren bevorzugten Ausführungsform der Erfindung kann oben in den allgemeinen chemischen Formeln (III) und (V) der Aminodiphenylsubstituent jeweils an zumindest einem Phenylring mono-, di- oder trisubstituiert sein, wobei der oder die Substituenten aus der bereits zuvor definierten Gruppe Z ausgewählt sein können.

Die erfindungsgemäß substituierten 2,2-Diaryl-2H-naphtho[1,2-b]pyrane und 3,3-Diaryl-3H-naphtho[2,1-b]pyrane zeigen in lichtangeregter Form gegenüber vergleichbaren bekannten Verbindungen eine bathochrom verschobene längstwellige Absorption im sichtbaren Teil des Spektrums. Diese überraschende Verschiebung tritt in gleicher Weise bei beiden genannten 2H- und 3H-Naphthopyransystemen auf.

Von großem Vorteil ist auch, daß die erfindungsgemäßen Verbindungen keine besondere basische Funktion im Molekül besitzen, da als Substituenten entweder cyclische Aminogruppen, wie Carbazol, Morpholin, Pyrrol, Pyrazol, Imidazol und dergleichen besonders bevorzugt sind, oder Aminogruppen geringer oder gar keiner Basizität, wie substituiertes oder unsubstituiertes Diphenylamin. Ganz besonders bevorzugt sind Verbindungen der Strukturformeln (III) bis (V) mit den genannten Substituenten.

Durch die gezielte Auswahl des Aminosubstituenten werden nachteilige Reaktionen bei der Verarbeitung dieser Farbstoffe und auch beim Langzeiteinsatz unter Lichteinwirkung generell ausgeschlossen. Die Lebensdauer der photochromen Farbstoffe wird somit wesentlich verlängert, es werden keine Protonen addiert, unerwünschte Farbabweichungen aufgrund von Nebenreaktionen erfolgen nicht.

Die Naphthopyransysteme der vorliegenden Erfindung zeigen insgesamt gute photochrome Reaktion hinsichtlich Eindunkelung und Aufhellung. Völlig überraschend ist dabei, daß diese Verbindungen im angeregten Zustand noch längerwelliger absorbieren als Verbindungen mit basischer Aminfunktion wie Dimethylamin. Überraschend ist auch der unerwartete visuelle Eindruck den die erfindungsgemäßen Verbindungen bewirken. Aufgrund der weit in den gelben Spektralbereich reichenden Absorption zeigen die Verbindungen der vorliegenden Erfindung trotz des nominell geringen λₘₐₓ-Wertes eine pinkfarbene bis violette Färbung. Dies ist in keiner Weise vorhersagbar und für derartige Verbindungen eine ungewöhnliche aus der Reihe fallende Eigenschaft.

Durch entsprechende Reaktionsführung konnten die mit Aminogruppen wie Morpholin, Piperidin etc. substituierten 2,2-Diaryl-2H-naphtho[1,2-b]pyrane bzw. 3,3-Diaryl-3H-naphtho[2,1-b]pyran-Verbindungen in reiner Form erhalten werden. Die Herstellung der erfindungsgemäßen Verbindungen wird nachfolgend anhand eines allgemeinen Reaktionsschemas kurz erläutert wobei als Aryl Phenylgruppen dargestellt sind und T Aminogruppen sind:

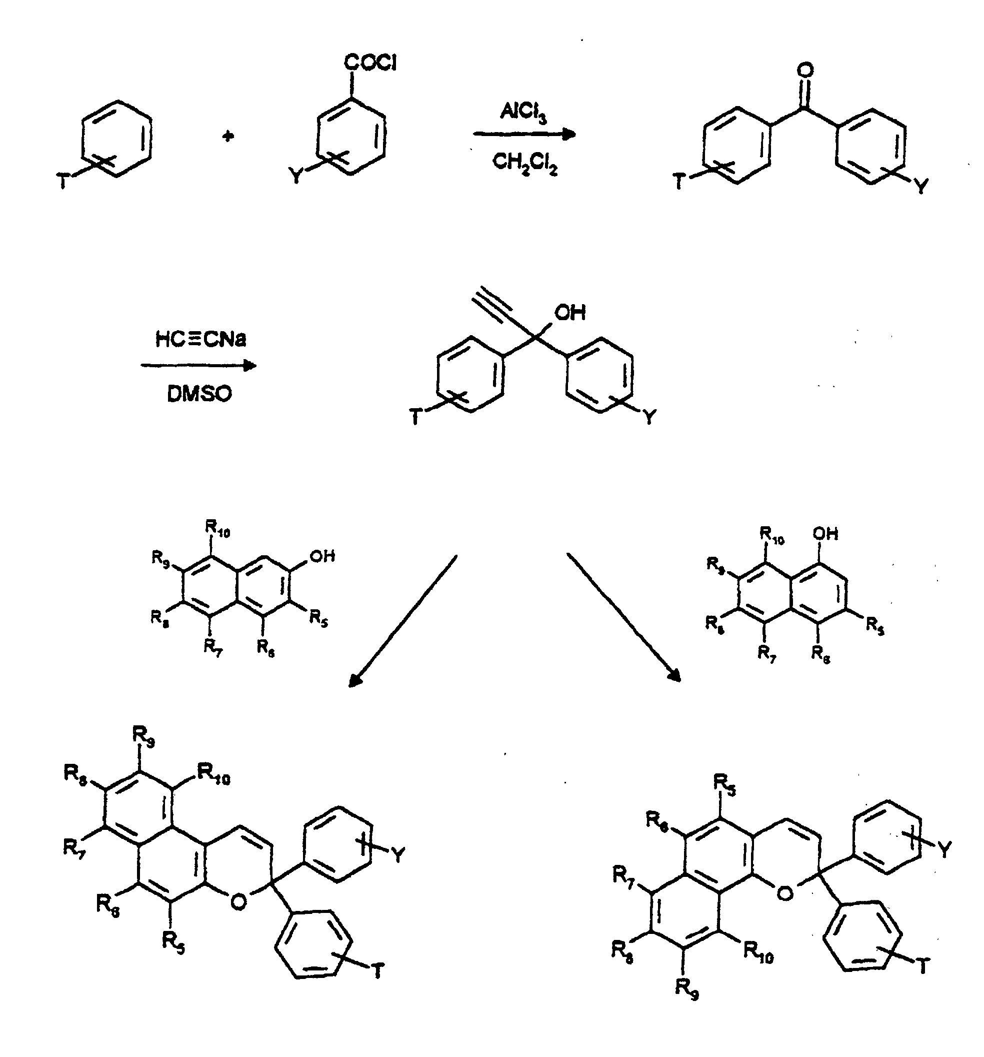

Die erfindungsgemäß bevorzugten, nichtbasischen Aminosubstituenten sind in besonders vorteilhafter Weise bei der Herstellung von photochromen Kunststoffgläsern verwendbar. Säurespuren, die bei der Hydrolyse des Gießharzes auftreten, attackieren diese Aminoverbindungen nicht und bewirken keine störenden Nebenreaktionen, wodurch der Verlust der photochromen Reaktion auftreten würde. Ebenso weisen die erfindungsgemäßen 2H- und 3H-Naphthopyranverbindungen eine im Vergleich deutlich bessere Lebensdauer auf als die aus dem Stand der Technik bekannten basischen Aminoverbindungen.

Die erfindungsgemäßen photochromen Naphthopyrane können generell in Kunststoffen aller Art Verwendung finden, insbesondere auch für ophthalmische Zwekke. Die Einsatzgebiete sind außerordentlich vielseitig und reichen von Linsen, Schutzgläsern, Visieren von Schutzhelmen, Fenstern, Brillengläsern bis zu Schutzblenden, Abdeckungen, Dächern oder dergleichen.

### Beispiele

Um die überraschenden Eigenschaften der erfindungsgemäßen Naphthopyrane zu demonstrieren, werden nachfolgend einige ausgewählte Verbindungen anhand ihrer längstwelligen Absorption λₘₐₓ charakterisiert und mit entsprechenden Vergleichsverbindungen verglichen. Es wurde hierfür vom Grundgerüst G eines 3,3-Diphenyl-3H-naphtho[2,1-b]pyrans ausgegangen, welches die folgende chemische Strukturformel besitzt:

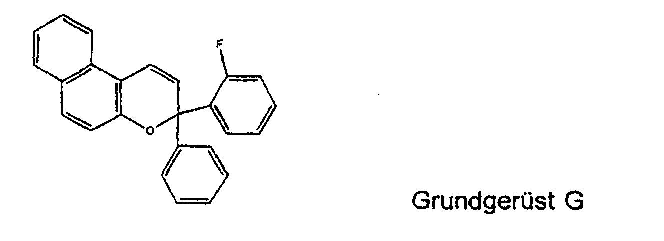

Es wurden im folgenden vier Verbindungen vermessen: Das Grundgerüst G, das am unsubstituierten Phenylring mit einer Methoxygruppe substituierte Grundgerüst G, sowie zwei erfindungsgemäße 3,3-Diphenyl-3H-naphtho[2,1-b]pyranverbindungen, wobei ein Phenylring mit Fluor und der andere Phenylring jeweils mit einer erfindungsgemäßen Aminogruppe, nämlich mit dem Morpholin-Rest (Gruppe (2)) und dem Diphenylaminorest (Gruppe (3)), substituiert wurde.

Die Messungen eraeben folgendes Bild:

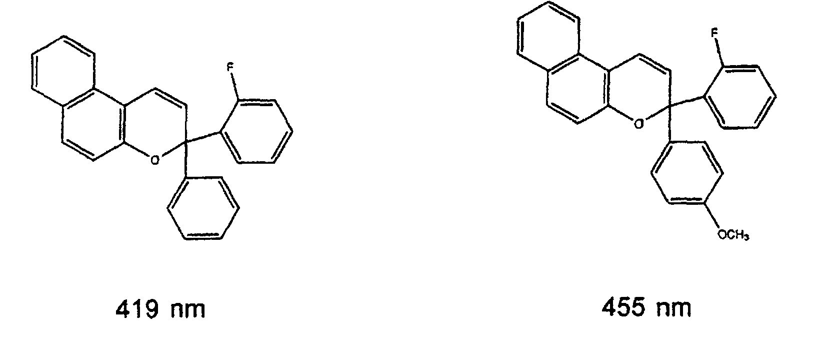

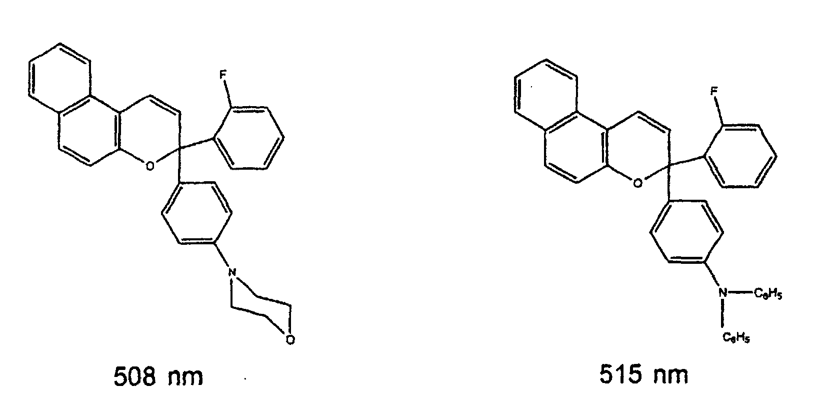

Das Grundgerüst G besitzt in Diglymelösung eine längstwellige Absorption knapp unter 420 nm. Substituiert man den zweiten (unsubstituierten) Phenylring mit einer elektronenabgebenden Gruppe, wie einer Methoxygruppe, so verschiebt sich λₘₐₓ auf 455 nm. Ein noch stärkerer Elektronendonator wie Morpholin verschiebt λₘₐₓ sogar auf 508 nm. Gemeinhin würde der Fachmann erwarten, daß eine weniger elektronenabgebende Gruppe wie Diphenylamin die längstwellige Absorption hypsochrom zu kürzeren Wellenlängen hin verschieben würde. Es wird jedoch genau der gegenteilige Effekt beobachtet, nämlich eine weitere bathochrome Verschiebung auf 515 nm.

### Herstellung der erfindungsgemäßen Verbindungen

Nachfolgend soll anhand einiger Beispiele die Herstellung der erfindungsgemäßen 2H- und 3H-Naphthopyranverbindungen erläutert werden:

### Beispiel 1

i) In einem 500 ml-Kolben werden 150 ml 1,2-Dichlorethan mit 16,1 g Aluminiumchlorid versetzt und unter Rühren und Kühlen mit Eiswasser 16,6 g 2-Fluorbenzoylchlorid zugetropft. Die Mischung wird ca. 15 min in Eiswasser gerührt, anschließend tropft man eine Lösung von 24,5 g Triphenylamin in 100ml 1,2-Dichlorethan zu. Man läßt die Mischung auftauen, erhitzt 1 h auf 50°C und läßt nach Abkühlen das Ganze über Nacht stehen. Am nächsten Tag wird auf 500 g Eiswasser gegossen. Nach ca. 10 min Rühren wird die organische Phase im Scheidetrichter abgetrennt und die wässrige Phase einmal mit 100 ml Dichlormethan extrahiert. Die vereinigten Extrakte werden mit Wasser, 5%iger Kalilauge und wieder mit Wasser gewaschen. Nach Trocknen über Natriumsulfat engt man die Lösung im Vakuum ein und chromatographiert an Aluminiumoxid (Wassergehalt 3%) mit einer Dichlormethan/Hexan-Mischung (1/4). Die hellgelben Kristalle (24 g) werden mittels NMR-Spektrum als 4-Diphenylamino-2'-fluor-benzophenon identifiziert.
ii) Das obige Reaktionsprodukt wird in 150 ml wasserfreiem Dimethylsulfoxid suspendiert, mit 4,7 g Natriumacetylid versetzt und über Nacht gerührt. Es entsteht eine braune Mischung, die auf 500 ml Eiswasser/200 ml Ether gegossen und unter Rühren mit 5 ml konzentrierter Salzsäure versetzt wird. Die organische Phase wird abgetrennt und die wäßrige Phase einmal mit 200 ml Ether extrahiert. Die vereinigten Etherphasen werden mit Wasser gewaschen und über Natriumsulfat getrocknet. Nach Abdestillieren des Lösungsmittels im Vakuum erhält man einen braunen viskosen Rückstand (24,3 g), der mittels NMR-Spektrum als 1-(4-Diphenylaminophenyl)-1-(2-fluorphenyl)-1-propinol identifiziert wurde.
iii) 5g des obigen Reaktionsproduktes werden mit 1,5 g 2-Naphthol in 100 ml Toluoi gelöst. Nach Zusatz einer Spatelspitze 4-Toluolsulfonsäure wird die Mischung 1 h bei 60°C gerührt. Nach Abkühlen wird das Solvens im Vakuum abdestilliert und der Rückstand , in 30 ml Dichlormethan gelöst, an Aluminiumoxid (Wassergehalt 3%) mit einer Dichlormethan/Hexan-Mischung (1/1) chromatographiert. Zur endgültigen Reinigung wird das Rohprodukt in etwa 100 ml Methanol digeriert, der Niederschlag abgesaugt und getrocknet. Das farblose Pulver (3,5 g) wurde mittels NMR-Spektrum als 3-(4-Diphenylaminophenyl)-3-(2-fluorphenyl)-3H-naphto[2,1-b]pyran identifiziert.

### Beispiel 2

Die Durchführung erfolgt analog Beispiel 1, nur im Schritt i) mit Phenylmorpholin (16,3 g) statt Triphenylamin und ohne Erhitzen der Reaktionsmischung. Es entsteht 2-Fluor-4-(N-morpholinyl)benzophenon (12,8 g), das analog Schritt ii) zu 1-(2-Fluorphenyl-1-[4-(N-morpholinyl)phenyl]-1-propinol (11,2 g) umgesetzt wird. Schritt iii) liefert 3-(2-Fluorphenyl)-3-[4-(N-morpholinyl)phenyl]-3H-naphtho[2,1-b]pyran (3,1g) als farbloses Pulver, das mittels NMR-Spektrum identifiziert wurde.

### Beispiel 3

Die Durchführung erfolgt analog Beispiel 1. Schritt i) erfolgt nach B. Staskun, J. Org. Chem. 1968, S.3031. Analog Schritt ii) entsteht 1-(4-Diphenylaminophenyl)-1-phenyl-1-propinol (19,8 g), das analog Schritt iii) mit 1-Naphthol statt 2-Naphthol umgesetzt wird. Der hellviolette Feststoff wurde mittels NMR-Spektrum als 2-(Diphenylaminophenyl)-2-phenyl-2H-naphtho[9,2-b]pyran (2,3 g) identifiziert.

## Patentansprüche

1. Photochrome Diaryl-Naphthopyrane mit den nachfolgenden allgemeinen chemischen Strukturformeln (I) oder (II): 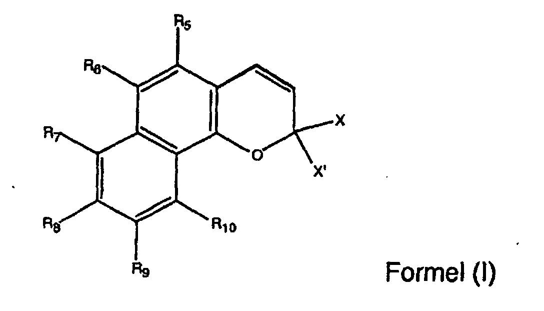 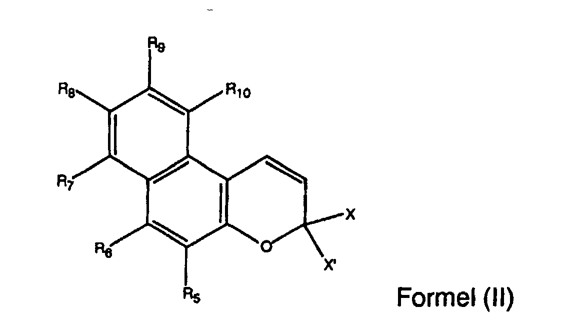 wobei
R₅ bis R₁₀ unabhängig voneinander gleich oder verschieden sind und aus der Gruppe Z, bestehend aus H, OH, C₁-C₁₀-Alkyl, C₁-C₆-Alkoxy, C₅-C₇-Cycloalkyl, Phenyl, Benzyl, Allyl, C₁-C₆-Alkoxy-( C₁-C₄)-Alkyl, Mono- oder Di-(C₁-C₆)-Alkyl substituiertem Phenyl, Furyl, Thienyl, Mono- oder Di-(C₁-C₆)-Alkoxy substituiertem Phenyl, C₁-C₆-Haloalkyl und Halogen ausgewählt sind, wobei das Halogen F, Cl oder Br und R₅ kein Wasserstoff ist;
X und X' unabhängig voneinander gleich oder verschieden sind und aus einer der folgenden Gruppen ausgewählt sind:
Gruppe (1): unsubstituierten, mono-, di- oder trisubstituierten Arylgruppen, Phenyl oder Naphthyl, wobei die Arylsubstituenten ausgewählt sind aus der Gruppe Z;
Gruppe (2): mono-, di- oder trisubstituierten Arylgruppen, Phenyl oder Naphthyl, wobei zumindest ein Substituent ausgewählt ist aus der Gruppe A, bestehend aus cyclischen Aminosubstituenten, wie Morpholin, Piperidin, Pyridin, Pyrrol, Pyrazol, Pyrazolidin, Piperazol, Indolin, Imidazol, Phenothiazin, Phenoxazin, Phenazin, Acridin, Carbazol, die unsubstituiert, mono-, di- oder trisubstituiert sind und der oder die Substituenten ausgewählt sind aus der Gruppe Z, und jeder weitere Substituent ausgewählt ist aus der Gruppe Z; oder
Gruppe (3): mono-, di- oder trisubstituierten Arylgruppen, Phenyl oder Naphthyl, wobei zumindest ein Substituent ausgewählt ist aus der Gruppe A', bestehend aus Aminodiphenyl, wobei ein oder zwei Phenylringe unabhängig voneinander unsubstituiert, mono-, di- oder trisubstituiert sind, und die Substituenten ausgewählt sind aus der Gruppe Z,
und entweder X oder X' aus einer der oben definierten Gruppen (2) oder (3) ausgewählt ist,
mit der Maßgabe, daß bei Verbindungen mit der Strukturformel (I) die cyclischen Aminosubstituenten Morpholin, Piperidin, 1-Indolinyl, Pyrrolidyl, 1-Imidazolidyl, 2-Imidazolin-1-yl, 2-Pyrazolidyl, Pyrazolinyl und 1-Piperazinyl der Gruppe (2) ausgeschlossen sind.

2. Photochrome Diaryl-Naphthopyrane mit den allgemeinen chemischen Strukturformeln (I) oder (II) nach Anspruch 1, wobei
R₅ bis R₁₀ unabhängig voneinander gleich oder verschieden und aus der in Anspruch 1 definierten Gruppe Z ausgewählt sind,
X oder X' ausgewählt ist aus der Gruppe, bestehend aus unsubstituierten, mono-, di- oder trisubstituierten Arylgruppen, Phenyl oder Naphthyl, wobei die Arylsubstituenten aus der Gruppe, bestehend aus OH, C₁-C₁₀-Alkyl, C₁-C₆-Alkoxy und Halogen ausgewählt sind; und das verbliebene X oder X' ausgewählt ist aus der Gruppe (2) oder (3) mit der Einschränkung von Anspruch 1.

3. Photochrome Diaryl-Naphthopyrane mit den allgemeinen chemischen Strukturformeln (I) oder (II) nach Anspruch 1, wobei
R₅ bis R₁₀ unabhängig voneinander gleich oder verschieden und aus der in Anspruch 1 definierten Gruppe Z ausgewählt sind,
und X und X' beide ausgewählt sind aus einer der Gruppen (2) oder (3) mit der Einschränkung von Anspruch 1.

4. Photochrome Diaryl-Naphthopyrane nach Anspruch 3, wobei X und X' aus der gleichen Gruppe ausgewählt sind.

5. Photochrome Diaryl-Naphthopyrane nach Anspruch 4, wobei X und X' gleich sind.

6. Photochrome Diaryl-Naphthopyrane nach einem der vorangehenden Ansprüche 1 bis 5, **dadurch gekennzeichnet, daß** eine Methoxygruppe in 8-Position des Naphthopyranrings vorhanden ist.

7. Photochrome 2,2-Diphenyl-2H-naphtho[1,2-b]pyrane mit der nachfolgenden chemischen Strukturformel (III): 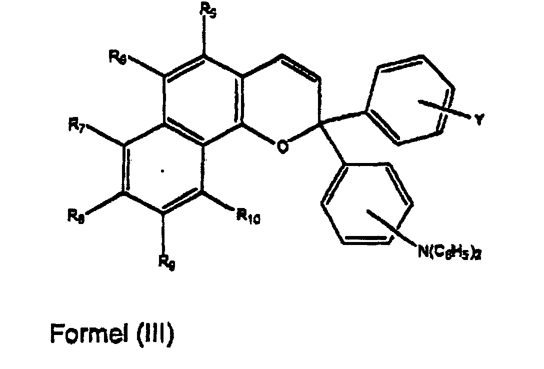 wobei
R₅ bis R₁₀ ausgewählt sind aus der in Anspruch 1 definierten Gruppe Z und
Y ausgewählt ist aus der Gruppe der Halogene F, Cl und Br.

8. Photochrome Diaryl-Naphthopyrane nach Anspruch 7, wobei der Aminodiphenylsubstituent in der chemischen Strukturformel (III) an zumindest einem Phenylring mono-, di- oder trisubstituieri ist und der oder die Substituenten ausgewählt sind aus der in Anspruch 1 definierten Gruppe Z.

9. Photochroms 3,3-Diphenyl-3H-naphtho[2,1-b]pyrane mit den nachfolgenden chemischen Strukturformeln (IV) oder (V): 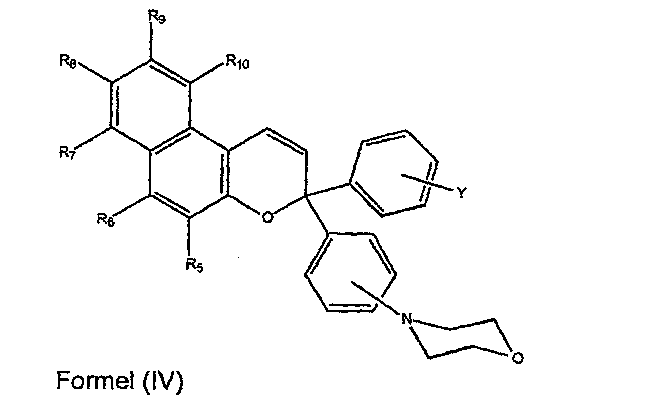 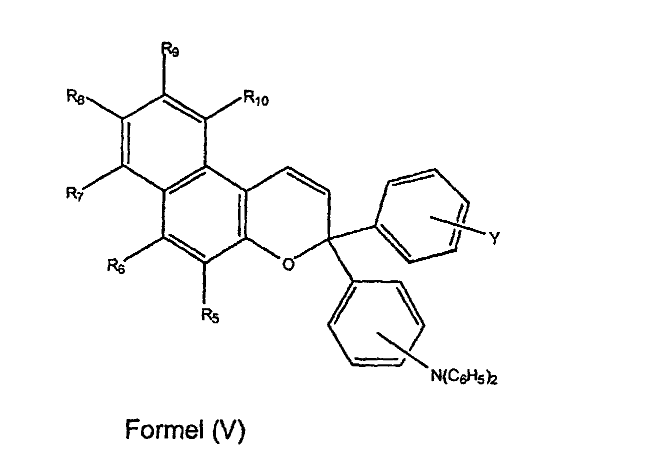 wobei
R₅ bis R₁₀ ausgewählt sind aus der in Anspruch 1 definierten Gruppe Z und
Y ausgewählt ist aus der Gruppe der Halogene F, Cl und Br.

10. Photochrome Diaryl-Naphthopyrane nach Anspruch 9, wobei der Aminodiphenylsubstituent in der chemischen Strukturformel (V) an zumindest einem Phenylring mono-, di- oder trisubstituiert ist und der oder die Substituenten ausgewählt sind aus der in Anspruch 1 definierten Gruppe Z.

11. Verwendung eines oder mehrerer der photochromen 2,2-Diaryl-2H- und/oder 3,3-Diaryl-3H-naphthopyrane nach einem der Ansprüche 1 bis 10 in Kunststoffen aller Art, insbesondere für ophthalmische Zwecke.

12. Verwendung eines oder mehrerer der photochromen 2,2-Diaryl-2H- und/oder 3,3-Diayyl-3H-naphthopyrane nach einem der Ansprüche 1 bis 10 in Kunststoffgegenständen, insbesondere Linsen, Schutzgläsem, Visieren von Schutzhelmen, Fenstern, Brillengläsern, Schutzblenden, Abdeckungen, Dächern oder dergleichen.

## Claims

1. Photochromic diaryl-naphthopyrans having the following general chemical structural formulae (I) or (II): 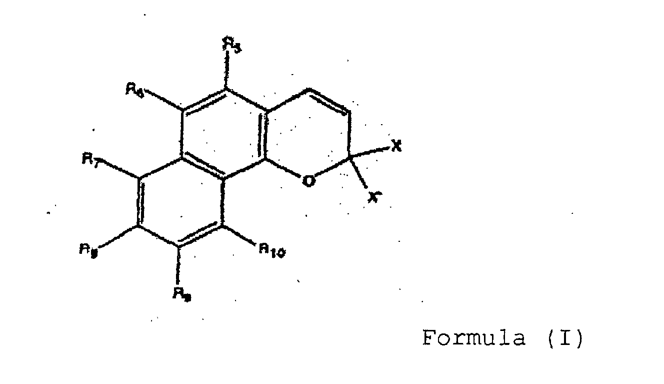 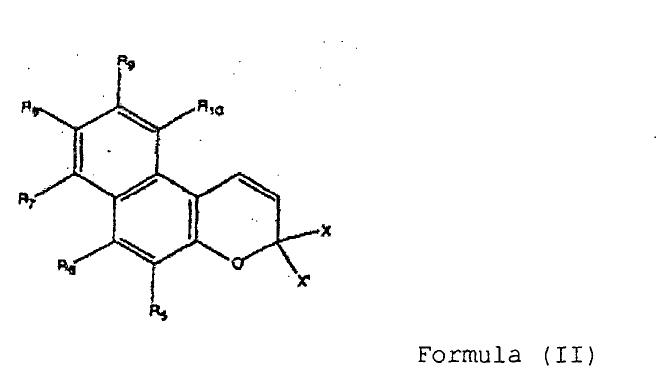 wherein
R₅ to R₁₀ independently of one another, are the same or different and are selected from the group Z, comprising H, OH, C₁-C₁₀ alkyl, C₁-C₆ alkoxy, C₅-C₇ cycloalkyl, phenyl, benzyl, allyl, C₁-C₆-alkoxy-(C₁-C₄)-alkyl, mono- or di-(C₁-C₆)-alkyl substituted phenyl, furyl, thienyl, mono- or di-(C₁-C₆)-alkoxy substituted phenyl, C₁-C₆ haloalkyl and halogen, the halogen being F, Cl or Br and R₅ not being hydrogen;
X and X' independently of one another, are the same or different and are selected from one of the following groups:
Group (1): unsubstituted, mono-, di- or trisubstituted aryl groups, phenyl or naphthyl, the aryl substituents being selected from group Z;
Group (2): mono-, di- or trisubstituted aryl groups, phenyl or naphthyl, at least one substituent being selected from the group A comprising cyclic amino substituents such as morpholine, piperidine, pyridine, pyrrole, pyrazole, pyrazolidine, piperazole, indoline, imidazole, phenothiazine, phenoxazine, phenazine, acridine, carbazole, which are unsubstituted, mono-, di- or trisubstituted and the substituent or substituents are selected from group Z, and each further substituent is selected from group Z; or
Group (3): mono-, di- or trisubstituted aryl groups, phenyl or naphthyl, at least one substituent being selected from the group A' comprising aminodiphenyl, one or two phenyl rings, independently of one another, being unsubstituted, mono-, di- or trisubstituted, and the substituents being selected from group Z,
and either X or X' is selected from one of the above-defined groups (2) or (3),
with the proviso that in the case of compounds having the structural formula (I), the cyclic amino substituents morpholine, piperidine, 1-indolinyl, pyrrolidyl, 1-imidazolidyl, 2-imidazoline-1-yl, 2-pyrazolidyl, pyrazolinyl and 1-piperazinyl of group (2) are excluded.

2. Photochromic diaryl-naphthopyrans having the general chemical structural formulae (I) or (II) according to claim 1, wherein
R₅ to R₁₀ independently of one another, are the same or different and are selected from the group Z defined in claim 1,
X or X' is selected from the group comprising unsubstituted, mono-, di- or trisubstituted aryl groups, phenyl or naphthyl, the aryl substituents being selected from the group comprising OH, C₁-C₁₀ alkyl, C₁-C₆ alkoxy and halogen;
and the remaining X or X' is selected from group (2) or (3) with the restriction of claim 1.

3. Photochromic diaryl-naphthopyrans having the general chemical structural formulae (I) or (II) according to claim 1, wherein
R₅ to R₁₀ independently of one another, are the same or different and are selected from the group Z defined in claim 1,
and X and X' are both selected from one of groups (2) or (3) with the restriction of claim 1.

4. Photochromic diaryl-naphthopyrans according to claim 3, wherein X and X' are selected from the same group.

5. Photochromic diaryl-naphthopyrans according to claim 4, wherein X and X' are the same.

6. Photochromic diaryl-naphthopyrans according to one of the preceding claims 1 to 5, **characterised in that** a methoxy group is present in position 8 of the naphthopyran ring.

7. Photochromic 2,2-diphenyl-2H-naphtho[1,2-b]pyrans having the following chemical structural formula (III) : 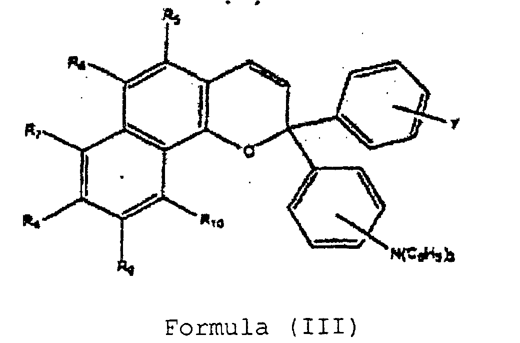 wherein
R₅ to R₁₀ are selected from the group Z defined it claim 1, and
Y is selected from the group of the halogens F, Cl and Br.

8. Photochromic diaryl-naphthopyrans according to claim 7, wherein the aminodiphenyl substituent in the chemical structural formula (III) is mono-, di- or trisubstituted at at least one phenyl ring and the substituent or substituents are selected from the group Z defined in claim 1.

9. Photochromic 3,3-Biphenyl-3H-naphtho[2,1-b]pyrans having the following chemical structural formulae (IV) or (V) : 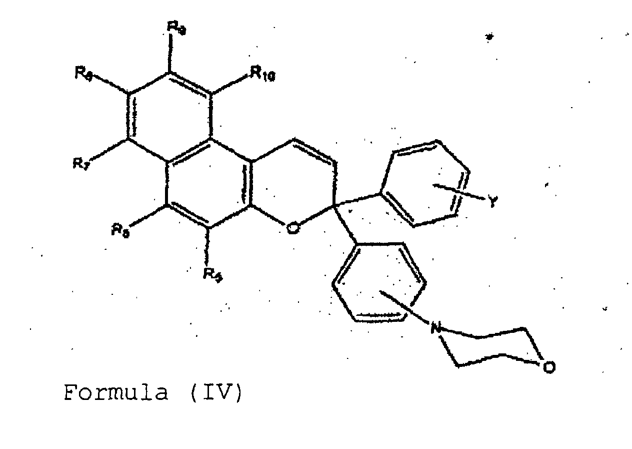 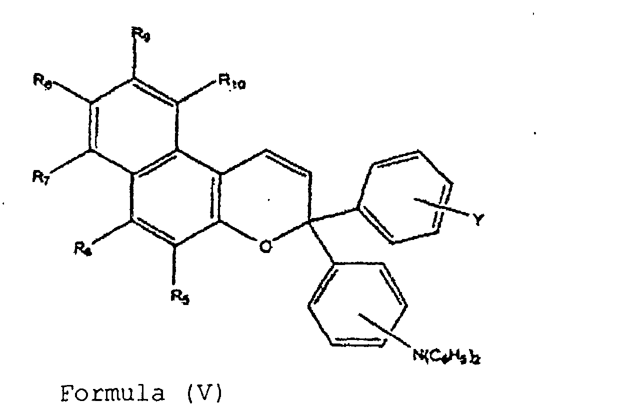 wherein
R₅ to R₁₀ are selected from the group Z defined in claim 1, and
Y is selected from the group of the halogens F, Cl and Br.

10. Photochromic diaryl-naphthopyrans according to claim 9, wherein the aminodiphenyl substituent in the chemical structural formula (V) is mono-, di- or trisubstituted at at least one phenyl ring, and the substituent or substituents are selected from the group Z defined in claim 1.

11. Use of one or more of the photochromic 2,2-diaryl-2H- and/or 3,3-diaryl-3H-naphthopyrans according to one of claims 1 to 10 in all kinds of plastics materials, especially for ophthalmic purposes.

12. Use of one or more of the photochromic 2,2-diaryl-2H and/or 3,3-diaryl-3H-naphthopyrans according to one of claims 1 to 10 in plastic objects, especially lenses, protective glasses, visors of protective helmets, windows, spectacle lenses, protective screens, coverings, roofs or the like.

## Revendications

1. Diaryl-naphtopyranes photochromes avec les formules générales de structure chimiques (I) ou (II) suivantes : 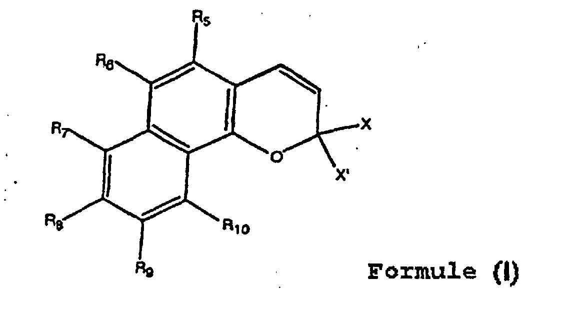 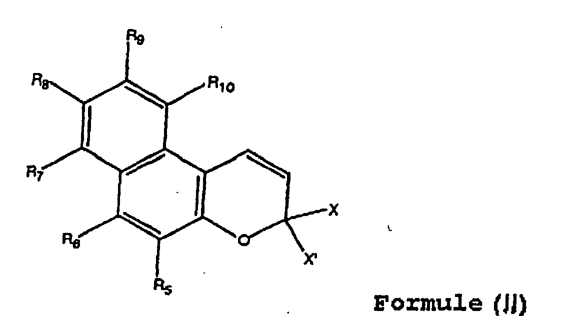 dans lesquels
R₅ à R₁₀ sont indépendamment l'un de l'autre identiques ou différents et sont choisis dans le groupe Z constitué de H, OH, alkyle en C₁ à C₁₀, alcoxy en C₁ à C₆, cycloalkyle en C₅ à C₇, phényle, benzyle, allyle, alcoxy en C₁ à C₆-alkyle en C₁ à C₄, phényle substitué par un mono- ou di-alkyle en C₁ à C₆, furyle, thiényle, phényle substitué par un mono- ou di-alcoxy en C₁ à C₆, haloalkyle en C₁ à C₆ et halogène, l'halogène étant F, Cl ou Br et R₅ n'étant pas un hydrogène ;
X et X' sont indépendamment l'un de l'autre identiques ou différents et sont choisis dans l'un des groupes suivants :
Groupe (1) : groupes aryle, phényle ou naphtyle non substitués, mono-, di- ou trisubstitués, où les substituants aryles sont choisis dans le groupe Z ;
Groupe (2) : groupes aryle, phényle ou naphtyle, mono-, di- ou trisubstitués, où au moins un substituant est choisi dans le groupe A constitué des substituants amino cycliques, comme la morpholine, la pipéridine, la pyridine, le pyrrole, la pyrazole, la pyrazolinidine, le pipérazole, l'indoline, l'imidazole, la phénothiazine, la phénoxazine, la phénazine, l'acridine, le carbazole, qui sont non substitués, mono-, di- ou trisubstitués et le ou les substituants étant choisis dans le groupe Z, et d'autres substituants étant choisis dans le groupe Z ; ou
Groupe (3) : groupes aryle, phényle ou naphtyle, mono-, di- ou trisubstitué, où au moins un des substituants est choisi dans le groupe A' constitué de l'aminodiphényle, dans lequel un ou deux noyaux phényle. sont indépendamment l'un de l'autre non substitués, mono-, di- ou trisubstitués, et les substituants sont choisis dans le groupe Z,
et X ou X' sont choisis dans l'un des groupes (2) ou (3) définis ci-dessus, sous réserve que dans les composés de formule développée (I) les substituants aminocycliques morpholine, pipéridine, 1-indolinyle, pyrrolidyle, 1-imidazolidyle, 2-imidazolin-1-yle, 2-pyrazolidyle, pyrazolinyle et 1-pipérazinyle du groupe (2) sont exclus.

2. Diaryl-naphtopyranes photochromes avec les formules générales de structure chimiques (I) ou (II) selon la revendication 1, dans lesquelles
R₅ à R_{1 0} sont indépendamment l'un de l'autre identiques ou différents et sont choisis dans le groupe Z défini dans la revendication 1,
X et X' sont choisis dans le groupe constitué par le groupe aryle, phényle ou naphtyle non substitué, mono-, di- ou trisubstitué, où les substituants aryle sont choisis dans le groupe constitué de OH, alkyle en C₁ à C₁₀, alcoxy en C₁ à C₆ et halogène, et le X ou X' restant est choisi dans le groupe (2) ou (3) avec la limitation de la revendication 1.

3. Diaryl-naphtyl pyranes photochromes avec les formules générales de strucutre chimiques (I) ou (II) selon la revendication 1, dans lesquelles
R₅ à R₁₀ sont indépendamment l'un de l'autre identiques ou différents et sont choisis dans le groupe Z défini dans la revendication 1,
et X et X' sont tous deux choisis dans l'un des groupes (2) ou (3) avec la limitation de la revendication 1.

4. Diaryl-naphtopyranes photochromes selon la revendication 3, dans lesquels X et X' sont choisis dans le même groupe.

5. Diaryl-naphtopyranes photochromes selon la revendication 4, dans lesquels X et X' sont identiques.

6. Diaryl-naphtopyranes photochromes selon l'une des revendication 1 à 5, **caractérisés en ce qu'**un groupe méthoxy est présent en position 8 du noyau naphtopyrane.

7. 2,2-diphényl-2H-naphto[1,2-b]-pyranes photochromes avec la formule chimique de structure (III) suivante : 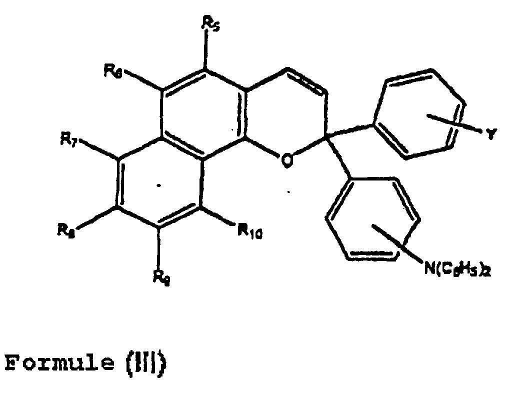 dans laquelle
R₅ à R₁₀ sont choisis dans le groupe Z défini dans la revendication 1 et
Y est choisi dans le groupe des halogènes F, Cl et Br.

8. Diaryl-naphtopyranes photochromes selon la revendication 7, dans lesquels le substituant diphényle dans la formule de structure chimique (III) est mono-, di- ou trisubstitué sur au moins un noyau phényle et le ou les substituants sont choisis dans le groupe Z défini dans la revendication 1.

9. 3,3-diphényl-3H-naphto[2,1-d]pyranes photochromes avec les formules de structure chimiques (IV) ou (V) suivantes : 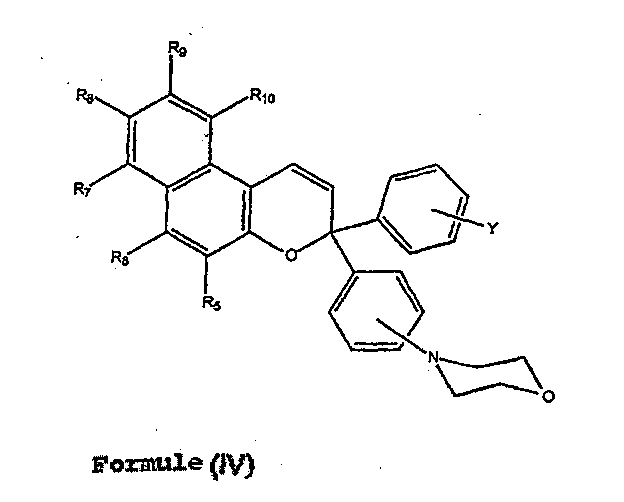 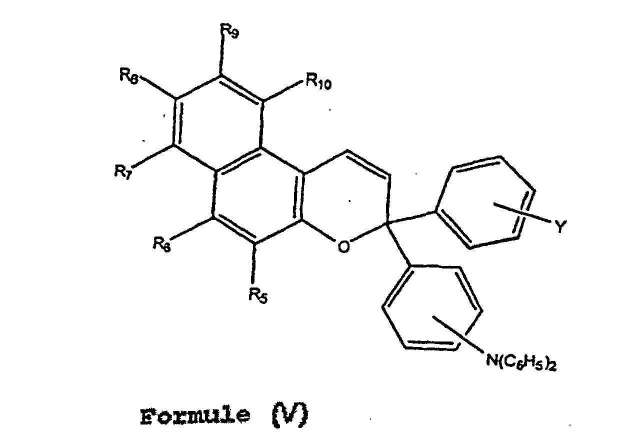 dans lesquelles R₅ à R₁₀ sont choisis dans le groupe Z défini dans la revendication 1 et
Y est choisi dans le groupe des halogènes, F, Cl et Br.

10. Diarylnaphtopyranes photochromes selon la revendication 9, dans lesquels le substituant aminodiphényle dans la formule de structure chimique (V) est mono-, di- ou trisubstitué sur au moins un noyau phényle et le ou les substituants sont choisis dans le groupe Z défini dans la revendication 1.

11. Utilisation d'un ou plusieurs des 2,2-diaryl-2H- et/ou 3,3-diaryl-3H-naphtopyranes photochromes selon l'une des revendications 1 à 10 dans des matières synthétiques de tous types, en particulier dans des buts ophtalmiques.

12. Utilisation d'un ou plusieurs des 2,2-diaryl-2H- et/ou 3,3-diaryl-3H-naphtopyranes photochromes selon l'une des revendications 1 à 10 dans des objets en matière synthétique, en particulier des lentilles, des verres protecteurs, des visières de casques protecteurs, des fenêtres, des verres de lunettes, des pare-brises, des revêtements, des toits ou analogues.
